## Europäisches Patentamt
## European Patent Office
## Office européen des brevets

(19)

(11) Veröffentlichungsnummer: **0 218 130**
**B1**

(12) **EUROPÄISCHE PATENTSCHRIFT**

(45) Veröffentlichungstag der Patentschrift:
**14.11.90**

(51) Int. Cl.⁵: **C12P 13/04, C12N 9/06**

(21) Anmeldenummer: **86112895.7**

(22) Anmeldetag: **18.09.86**

(54) Verfahren zur fermentativen Herstellung von L-Aminosäuren aus alpha-Ketocarbonsäuren.

(30) Priorität: **18.09.85 DE 3533198**

(43) Veröffentlichungstag der Anmeldung:
**15.04.87 Patentblatt 87/16**

(45) Bekanntmachung des Hinweises auf die Patenterteilung:
**14.11.90 Patentblatt 90/46**

(84) Benannte Vertragsstaaten:
**BE CH DE FR GB LI NL SE**

(73) Patentinhaber: **Forschungszentrum Jülich GmbH,**
**Postfach 1913, D-5170 Jülich(DE)**
Patentinhaber: **Degussa Aktiengesellschaft,**
**Weissfrauenstrasse 9, D-6000 Frankfurt am Main 1(DE)**

(72) Erfinder: **Mütze, Bernhard, Dr., Auf dem Berg 3,**
**D-5170 Jülich(DE)**
Erfinder: **Ohshima, Toshihisa, Prof. Dr., Kyoto University**
**of Education Depart. of Chemistry, Fushimi-ku**
**Kyoto 612(JP)**
Erfinder: **Wandrey, Christian, Prof. Dr., Wolfshovener**
**Strasse 139, D-5170 Jülich(DE)**
Erfinder: **Leuchtenberger, Wolfgang, Dr.,**
**Geschwister-Scholl-Strasse 1, D-6454 Bruchköbel(DE)**

(56) Entgegenhaltungen: (Fortsetzung)
Ohio, US; C. WANDREY et al.: "Multienzyme systems in membrane reactors", & ENZYME ENG. 1982, 6, 61-7
CHEMICAL ABSTRACTS, Band 85, 1976, Seite 255, Zusammenfassung Nr. 174086c, Columbus, Ohio, US; I. EPSTEIN et al.: "Regulatory control and function of alanine dehydrogenase form a thermophilic bacillus", & BIOCHIM. BIOPHYS.
ACTA 1976, 445(3), 549-
57 P., 3rd 1982 (PUB. 1983), 273-84 000
"BERGEY'S MANUAL OF DETERMINATIVE BACTERIOLOGY", 8. Ausgabe, 1974, Seite 550, The Williams & Wilkins Co., US;
"THE MICROBIAL PRODUCTION OF AMINO ACIDS", 1972, Seiten 326-333, Tokyo, JP;
CHEMICAL ABSTRACTS, Band 104, 1986, Seite 389, Zusammenfassung Nr. 65540m, Columbus, Ohio, US; T. OHSHIMA et al.: "Screening of thermostable leucine and alanine dehydrogenases in thermophilic bacillus strains", & BIOTECHNOL.
LETT. 1985, 7(12), 871-6P., 3rd 1982 (PUB. 1983), 273-84 000

(56) Entgegenhaltungen:
EP-A- 0 101 653
EP-A- 0 140 503

PATENTS ABSTRACTS OF JAPAN, Band 8, Nr. 122 (C-227)[1559], 8. Juni 1984; & JP-A-59 34 890 (DAIICHI KAGAKU YAKUHIN K.K.) 25-02-1984
IDEM
CHEMICAL ABSTRACTS, Band 100, Nr. 17, April 1984, Seite 501, Zusammenfassung Nr. 137282w, Columbus, Ohio, US; E. FIOLITAKIS et al.: "Reaction technology of the enzymically catalyzed production of L-alanine", & ENZYME TECHNOL., ROTENBURG FERMENT. SYMP., 3rd 1982 (PUB. 1983), 273-84 000
CHEMICAL ABSTRACTS, Band 98, Nr. 7, Februar 1983, Seite 517, Zusammenfassung Nr. 51907g, Columbus,

## Beschreibung

Die Erfindung bezieht sich auf ein Verfahren zur Herstellung von L-Aminosäuren aus den entsprechenden α-Ketocarbonsäuren durch bakterielle Fermentation in Gegenwart von Ammoniumionen.

Die Herstellung von L-Aminosäuren aus α-Ketocarbonsäuren auf mikrobiologischem Wege ist z. B. aus der JP-A-59 34 890 oder aus der DE-OS 34 27 495 bekannt, in der die Herstellung von L-Aminosäuren aus den entsprechenden Ketocarbonsäuren durch Einwirkung von Glutaminsäure ausscheidenden Bakterien insbesondere aus der Gattung Brevibacterium und Corynebacterium oder Eschercia coli beschrieben wird. Dabei wird insbesondere die logarithmische Wachstumsphase der Mikroorganismen für die Umwandlung ausgenutzt und so bei Temperaturen in der Gegend von 30 - 37°C und α-Ketocarbonsäurekonzentrationen in der Kulturbrühe von etwa 20 - 50 g/l und Zeiten zwischen 20 und 72 Stunden eine bis zu 100 %-ige Umwandlung der Ketosäure erreicht.

Bei einem solchen Fermentationsverfahren muß darauf geachtet werden, daß Fremdkeime ausgeschlossen werden, die in Nährstoffkonkurrenz zum Kulturorganismus treten, das gewünschte Produkt verwerten oder andere biologische Störungen verursachen können, was zu besonderer Sorgfalt zwingt.

Es wurde nun gefunden, daß die Fremdkeimgefahr vermindert und zudem hohe Raum/Zeit-Ausbeuten sowie zusätzliche Verfahrensvorteile erzielt werden können, wenn die mikrobiologische Umsetzung von α-Ketocarbonsäuren in die entsprechenden L-Aminosäuren bei erhöhten Temperaturen mit Hilfe von thermophilen Bacillus-Stämmen bei einer Temperatur über 45°C durchgeführt wird.

Das erfindungsgemäße Verfahren der eingangs genannten Art ist demgemäß dadurch gekennzeichnet, daß die Fermentation mit Hilfe von thermophilen Bacillus-Stämmen bei einer Temperatur über 45°C durchgeführt wird.

Vorzugsweise erfolgt die Umsetzung bei Temperaturen über 60°C.

Die erfindungsgemäße reduktive Aminierung von α-Ketosäuren bei erhöhten Temperaturen hat neben der Verminderung der Kontaminationsgefahr bei der Fermentation durch Fremdkeime den Vorteil, daß die Viskosität des Fermenterinhaltes sinkt, wodurch höhere Filtrationsleistungen bei der Biomasserückhaltung und niedrigere Energiekosten für das Rühren und Pumpen erzielt werden können. Ferner wird die Löslichkeitsgrenze der gebildeten Aminosäuren heraufgesetzt, wodurch in Lösung höhere Produktkonzentrationen erreichbar sind, was eine Isolierung der erhaltenen L-Aminosäuren, wie weiter unten näher beschrieben ist, erleichtert.

Zwar ist die mikrobielle Gewinnung von Aminosäuren bei erhöhter Temperatur mit thermophilen Bakterien seit langem bekannt, wie z. B. die Bildung von DL-Alanin aus Glucose bei 50 - 65°C mit Bacillus coagulans B₉-17 (Agr. Biol. Chem. 31 (1967) 1381 - 1388), jedoch war die spezielle Nützlichkeit der Umsetzung von α-Ketocarbonsäure zu der entsprechenden L-Aminosäure bei erhöhter Temperatur mit Hilfe von thermophilen Bakterien bislang offensichtlich nicht erkannt.

Das erfindungsgemäße Verfahren ist insbesondere für die Bildung von Valin aus α-Ketoisovaleriat, Leucin aus α-Ketoisocaproat, Isoleucin aus α-Keto-β-methylvaleriat, Alanin aus Pyruvat und Phenylalanin aus Phenylpyruvat anwendbar.

Als thermophile Bacillen kommen z.B.

| Bacillus caldothenax | DSM | 406 |
| Bacillus spec. | DSM | 411 |
| | " | 405 |
| | " | 465 |
| | " | 466 |
| | " | 1519 |
| | " | 1520 |
| | " | 1521 |
| | " | 730 |
| Bacillus sphaericus | DSM | 461 |
| | " | 462 |
| | " | 463 |
| Bacillus stearothermophilus | DSM | 458 |
| | " | 1550 |
| Bacillus coagulans | DSM | 460 |
| | " | 2320 |
| Bacillus acidocaldarius | DSM | 452 |

in Betracht, die insbesondere für die Bildung von Leucin und Alanin geeignet sind. Untersucht wurden speziell Bacillus acidocaldarius DSM 452 (Hinterlegungsnummer des bei der Deutschen Sammlung von Mikroorganismen in Göttingen, BRD, hinterlegten Bacillus-Stamms) Bacillus sp. DSM 465 und DSM 466, Bacillus sphaericus DSM 461, 462 und 463 sowie Bacillus caldothenax DSM 406, die sich zur Umwandlung von α-Keto-isocaproat in L-Leucin und Pyruvat zu Alanin als besonders zweckmäßig erwiesen haben.

Als zusätzliche Energiequelle konnten mit Erfolg billige C-Quellen wie Acetat und Glycerin als Co-Substrat eingesetzt werden. Dabei kann das verbrauchte Acetat besonders leicht durch pH-geregelte Zudosierung von Essigsäure ersetzt werden. Durch Verwendung von Acetat kann der Glycolyseweg umgangen werden.

Die Fermentation kann im Batch-Verfahren oder kontinuierlich durchgeführt werden. Durch Biomasserückhaltung bei der kontinuierlichen Fermentation mit Hilfe von Filtersystemen oder durch Zentrifugieren wird erreicht, daß aus dem Fermenter überwiegend erschöpftes Medium mit dem Produkt abläuft, während die Mikroorganismen ganz oder zum Teil in den Fermenter zurückgeführt werden können. Besonders zweckmäßig kann in einem Durchlaufreaktor gearbeitet werden. Aus dem Kulturfiltrat wird in einem Kristallisator die Aminosäure durch Abkühlen gewonnen, und die Mutterlauge wird ganz oder teilweise in den Reaktor zurückgeführt.

Die bei erhöhter Temperatur günstige rasche Umwandlung der α-Ketocarbonsäure in die entsprechende L-Aminosäure erlaubt die Gewinnung eines Fermenterablaufs mit geringer Ketosäurekonzentration und hoher Aminosäurekonzentration, so daß die Ausscheidung und Gewinnung der Aminosäure einfach durch entsprechende Abkühlung (etwa bis herab zu +2°C) erreicht werden kann.

Besonders interessant ist eine gewisse Drosselung der $O_2$-Zufuhr zum Fermenter, auf weniger als 20% gelöst-$O_2$, wodurch die Produktausbeute überraschenderweise erhöht werden kann. So wurde beispielsweise bei der Umwandlung von α-Keto-isocaproat in L-Leucin mit Hilfe von Bacillus caldothenax bei 60°C und Na-isocaproat-Konzentrationen von 7,7 g/l durch Verminderung der Belüftung von 1 V/V min auf 0,05 V/V min eine Steigerung der Selektivität von 50% auf 80% erzielt.

Nachfolgend wird die Erfindung anhand von Beispielen mehr im einzelnen erläutert:

Beispiel 1

Gewinnung von L-Leucin aus α-Ketoisocaproat und Ammonium mit Bacillus caldothenax (DSM 406) in einer Batch-Fermentation

Für die Fermentation wurde folgendes Medium verwendet: Pepton 1 g; Hefeextrakt 0,5 g; Fleischextrakt 0,5 g; Na-Acetat 6 g; $K_2HPO_4$ 2 g; $KH_2PO_4$ 2 g; $Mg\_SO_4$ 0,1 g; $NH_4Cl$ 10,65 g; α-Ketoisocaproat 7,67 g; ad 1 l entionisiertes Wasser; pH 7,2. Autoklaviert wurde bei + 120°C 20 Min.

Der Fermenterinhalt betrug 3 l, die Temperatur lag bei + 60°C. Der pH-Wert wurde durch Zudosieren von 100%iger Essigsäure auf 7,2 geregelt. Auf diese Weise wurde das verbrauchte Acetat ersetzt. Gerührt wurde mit 800 U/min. Der Fermenter wurde mit einer dichten Suspension von Bacillus caldothenax beimpft, der auf einem Fleischextrakt-Hefeextrakt-Pepton-Medium angezüchtet worden war. Zur Vermeidung einer Substratinhibierung wurden Ketosäure und Ammoniumsalz sukzessive ergänzt. Dabei wurde eine L-Leucin-Konzentration von 17 g/l erreicht. Bei hoher Belüftungsrate von 1 V/V min wurden 50% der verbrauchten Ketosäure in L-Leucin umgewandelt, bei 0,05 V/V min dagegen 80%.

Beispiel 2

Gewinnung von L-Leucin aus α-Ketoisocaproat und Ammonium mit Bacillus caldothenax in einer kontinuierlichen Fermentation mit Biomasse-Rückhaltung und kontinuierlicher Produkt-Ernte.

Für diese Fermentation wurde des Medium des Beispiels 1 verwendet. Das Fermenter-Volumen betrug 3 l. Ketosäure und Ammoniumsalz wurden getrennt kontinuierlich zugeführt. Das verbrauchte Acetat wurde durch Essigsäure ersetzt, die pH-gesteuert zudosiert wurde. Der pH-Wert lag bei 7,2, die Temperatur bei + 60°C. Beimpft wurde wie in Beispiel 1. Die Luftzufuhr betrug zunächst 1 V/V min, um durch schnelles Wachstum eine hohe Zellkonzentration zu erreichen. Danach wurde die Luft wiederum auf 0,05 V/V min herabgesetzt, um die L-Leucinproduktion wie in Beispiel 1 zu steigern. Aus dem Fermenterablauf wurde die Biomasse durch ein Filtersystem zurückgehalten und in den Fermenter zurückgepumpt. Das Filtrat wurde zur Aminosäureausscheidung im Kristallisator abgekühlt. Die Mutterlauge wurde teilweise in den Fermenter zurückgeführt. Der andere Teil lief ab und wurde kontinuierlich durch neues Medium ersetzt. Durch die Kühlung kristallisierte L-Leucin aus und wurde kontinuierlich oder diskontinuierlich abgepumpt.

Durch die hohe Fermentationstemperatur von 60°C ist die Sättigungskonzentration des L-Leucins erhöht und im Fermenter kann mehr Produkt in Lösung gehalten werden. Durch Kühlung kann damit mehr L-Leucin ausgeschieden werden als aus der Fermenterbrühe einer Fermentation bei niedrigeren Temperaturen. Die geringere Viskosität des Fermenterinhaltes bei 60°C erhöhte die Filtrationsleistung und reduzierte den Energieaufwand für die Filtration.

Die Verweilzeit τ lag bei diesem Versuch bei 24 Std. Pro Tag und Liter Fermenterinhalt wurden 2,5 g L-Leucin gebildet, wobei 75% der verbrauchten Ketosäure in L-Leucin umgewandelt wurden. Die Filterfläche des Kreuzstrom-Mikrofilters der Firma Enka lag bei 0,036 m². Zur Vermeidung einer Deckschichtbildung wurde die Strömungsgeschwindigkeit parallel zur Filterfläche auf 1 m/s eingestellt. Durch Nährstofflimitierung und Rückführung aller Zellen in den Fermenter konnte L-Leucin auch mit ruhenden Zellen kontinuierlich produziert werden.

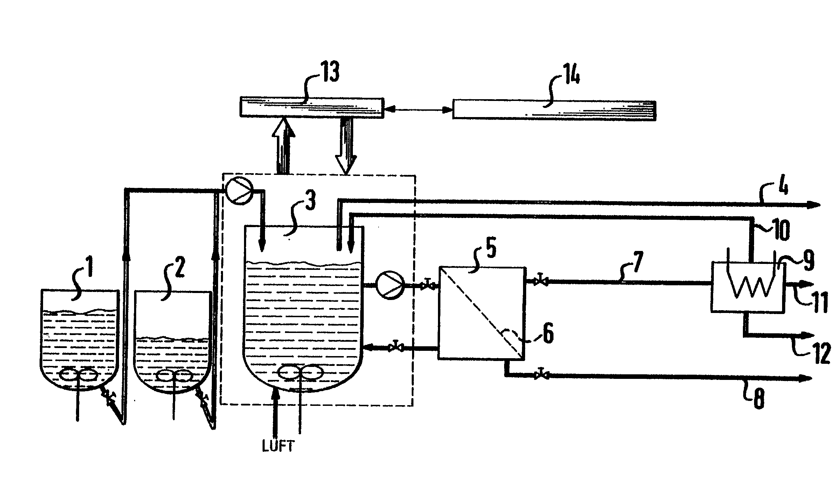

Die beigefügte Figur zeigt ein Schema für eine Vorrichtung zur Durchführung des Verfahrens: Aus den Substratsterilisiergefäßen 1 und 2 wird Substrat in den belüfteten Fermenter 3 dosiert, aus dem die Abluft über 4 entweicht. Die belüftete und gerührte Fermenterbrühe wird kontinuierlich zu einem Separator 5 geleitet, der ein Filtersystem 6 zur Biomasserückhaltung aufweist. Die von Biomasse befreite Produktlösung wird über 7 abgegeben, während Produktlösung mit Biomasse über 8 abgezogen werden kann.

Die vom Separator 5 über 7 abgezogene Produktlösung gelangt zum Kristallisator 9. Die Mutterlauge wird teilweise über 10 in den Fermenter 3 zurückgeführt. Ein Mutterlauge-Bleed-Strom geht über 11 ab. Produkt wird bei 12 abgegeben.

Der Betrieb des Fermenters 3 wird über ein Regelsystem 13 mit Prozeßrechner 14 gesteuert.

Beispiel 3

Gewinnung von L-Alanin aus Pyruvat mit den thermophilen Bacillus-Spezies DSM 465 und 466.

Für die Fermentation wurde das in Beispiel 1 angegebene Medium mit 8 g/l Brenztraubensäure-Na-Salz (Pyruvat) an Stelle von α-Ketoisocaproat verwendet. Statt Acetat wurde auch wahlweise Glycerin verwendet. Die Fermentationsbedingungen entsprachen denen von Beispiel 1.

Bei einem gelöst-Sauerstoffgehalt von 10% wurden 80% des vorgelegten Pyruvats bei 60°C in L-Alanin umgewandelt. Es entstanden 5,3 g L-Alanin/l xTag.

**Patentansprüche**

1. Verfahren zur Herstellung von L-Aminosäuren aus den entsprechenden α-Ketocarbonsäuren durch bakterielle Fermentation in Gegenwart von Ammoniumionen,
**dadurch gekennzeichnet,**
daß die Fermentation mit Hilfe von thermophilen Bacillus-Stämmen bei einer Temperatur über 45°C durchgeführt wird.

2. Verfahren nach Anspruch 1,
**dadurch gekennzeichnet,**
daß die Fermentation bei Temperaturen über 60°C durchgeführt wird.

3. Verfahren nach Anspruch 1 oder 2,
**dadurch gekennzeichnet,**
daß L-Leucin oder L-Alanin aus α-Keto-isocaproat oder Pyruvat hergestellt werden.

4. Verfahren nach einem der vorangehenden Ansprüche,
**dadurch gekennzeichnet,**
daß Bacillus acidocaldarius DSM 452, Bacillus sp. DSM 465 oder DSM 466, Bacillus sphaericus DSM 461, 462 oder 463 oder insbesondere Bacillus caldothenax DSM 406 als thermophile Bakterien verwendet werden.

5. Verfahren nach einem der vorangehenden Ansprüche,
**dadurch gekennzeichnet,**
daß aus dem Fermenter kontinuierlich Fermentationsflüssigkeit entnommen und unter Ausscheidung und Gewinnung von Aminosäure abgekühlt und anschließend rückgepumpt wird.

6. Verfahren nach einem der vorangehenden Ansprüche,
**dadurch gekennzeichnet,**
daß die Fermentation in einem kontinuierlich betriebenen Fermenter unter Biomasserückhaltung erfolgt und unter Recyclierung von Ablauf, der nach Kühlung und Ausscheidung von L-Aminosäure in den Fermenter zurückgegeben wird.

7. Verfahren nach einem der vorangehenden Ansprüche,
**dadurch gekennzeichnet,**
daß als Co-Substrate Acetat und/oder Glycerin und/oder Glucose verwendet werden.

8. Verfahren nach einem der vorangehenden Ansprüche,
**dadurch gekennzeichnet,**
daß die O₂-Zufuhr zum Fermenter auf einen Wert von weniger als 20% gelöst -O₂ gedrosselt wird.

**Claims**

1. Process for the production of L-amino acids from the corresponding α-keto acids by bacterial fermentation in the presence of ammonium ions, characterized in that the fermentation is carried out at a temperature of above 45°C with the aid of thermophilic Bacillus strains.

2. Process according to Claim 1, characterized in that fermentation is carried at temperatures of above 60°C.

3. Process according to Claim 1 or 2, characterized in that L-leucine or L-alanine are prepared from α-ketoisocaproate or pyruvate.

4. Process according to one of the preceding claims, characterized in that Bacillus acidocaldarius DSM 452, Bacillus sp. DSM 465 or DSM 466, Bacillus sphaericus DSM 461, 462 or 463 or, in particular, Bacillus caldothenax DSM 406 are used as thermophilic bacteria.

5. Process according to one of the preceding claims, characterized in that fermentation liquid is removed continuously from the fermenter and is cooled, with precipitation and isolation of amino acid, and is then pumped back.

6. Process according to one of the preceding claims, characterized in that the fermentation is carried out in a fermenter which is operated continuously with retention of the biomass and with recycling of the discharge, which is fed back into the fermenter after cooling and precipitation of L-amino acid.

7. Process according to one of the preceding claims, characterized in that acetate and/or glycerol and/or glucose are used as co-substrates.

8. Process according to one of the preceding claims, characterized in that the O₂ supply to the fermenter is reduced to a value of less than 20% dissolved O₂.

**Revendications**

1. Procédé de préparation des L-acides aminés à partir des acides α-cétocarboxyliques correspondants, par fermentation bactérienne en présence d'ions ammonium, caractérisé, en ce qu'il consiste à effectue la fermentation à l'aide de bacilles thermophiles à une température supérieure à 45°C.

2. Procédé suivant la revendication 1, caractérisé, en ce qu'il consiste à effectuer la fermentation à des températures supérieures à 60°C.

3. Procédé suivant la revendication 1 ou 2, caractérisé, en ce qu'il consiste à préparer de la leucine lévogyre ou de l'alanine lévogyre à partir d'α-cétoisocaproate ou de pyruvate.

4. Procédé suivant l'une des revendications précédentes, caractérisé, en ce qu'il consiste à utiliser comme bactéries thermophiles, Bacillus acidocaldarius DSM 452, Bacillus sp. DSM 465 ou DSM 466, Bacillus sphaericus DSM 461, 462 ou 463 ou, notamment, Bacillus caldothenax DSM 406.

5. Procédé suivant l'une des revendications précédentes, caractérisé, en ce qu'il consiste à prélever en continu du fermenteur du liquide de fermentation que l'on refroidit, tout en séparant et en obtenant de l'acide aminé, et que l'on retourne ensuite par pompage.

6. Procédé suivant l'une des revendications précédentes, caractérisé, en ce qu'il consiste à effectuer la fermentation dans un fermenteur fonctionnant en continu, tout en retenant la biomasse et en recyclant le résidu que l'on retourne, après refroidissement et séparation de l'acide aminé lévogyre, au fermenteur.

7. Procédé suivant l'une des revendications précédentes, caractérisé, en ce qu'il consiste à utiliser, comme cosubstrat, de l'acétate et/ou de la glycérine et/ou du glucose.

8. Procédé suivant l'une des revendications. précédentes, caractérisé, en ce qu'il consiste à réduire l'arrivée de $O_2$ au fermenteur à une valeur inférieure à 20 % de $O_2$ à l'état dissous.

LUFT